# FASCICULE DE BREVET EUROPEEN

(11) **EP 2 696 765 B1**
(45) Date de publication et mention de la délivrance du brevet: **02.03.2016**
(21) Numéro de dépôt: 12724662.7
(22) Date de dépôt: 13.04.2012
(51) Int. Cl.: A61B 5/15, A61B 17/34, A61M 25/01

(54) **DISPOSITIF D'AIDE AU PRÉLÈVEMENT DE LIQUIDE BIOLOGIQUE PRÉSENT DANS UNE CAVITÉ**
VORRICHTUNG ZUR UNTERSTÜTZUNG DER PROBENENTNAHME AUS EINER BIOLOGISCHEN FLÜSSIGKEIT IN EINEM HOHLRAUM
DEVICE FOR ASSISTING WITH THE SAMPLING OF A BIOLOGICAL FLUID PRESENT IN A CAVITY

(30) Priorité: 14.04.2011 FR 1153229
(43) Date de publication de la demande: 19.02.2014
(73) Titulaire: Assistance Publique Hôpitaux De Paris, 75004 Paris 4 (FR)
(72) Inventeur: BENBUNAN, Marc, F-75003 Paris (FR)
(74) Mandataire: Lebkiri, Alexandre
(86) Numéro de dépôt international: PCT/FR2012/050817
(87) Numéro de publication internationale: WO 2012/140382

(56) Documents cités:
- EP-A1- 0 137 528
- EP-A2- 0 931 559
- WO-A1-2007/035482
- WO-A1-2007/103999
- WO-A2-2009/076188
- US-A- 4 083 370
- US-A- 4 986 810
- US-A- 5 637 097

## Description

### DOMAINE TECHNIQUE DE L'INVENTION

La présente invention concerne un dispositif d'aide au prélèvement de liquide biologique dans une cavité, et plus particulièrement, mais non exclusivement, de sang placentaire dans un cordon ombilical. Le dispositif est préférentiellement destiné aux cas dans lesquels la cavité est un vaisseau non innervé tel qu'un cordon ombilical ou bien un tube synthétique utilisé en laboratoire. En effet, un prélèvement utilisant un tel dispositif est douloureux.

Le domaine technique est, d'une façon générale, celui des dispositifs utilisés pour effectuer des prélèvements de liquide biologique. Le dispositif selon l'invention est apte à coopérer avec un dispositif de prélèvement de liquide biologique. L'invention sera préférentiellement décrite dans le cas où le dispositif de prélèvement de liquide biologique est une aiguille de ponction classique.

### ARRIERE PLAN TECHNOLOGIQUE DE L'INVENTION

Prélever du sang placentaire provenant de cordons ombilicaux est une pratique effectuée de façon croissante ces dernières années, dans le cadre de greffes de cellules souches hématopoïétiques comme alternative au prélèvement de moelle osseuse, pour reconstituer l'hématopoïèse chez des patients souffrant de maladies hématologiques. Actuellement, pour effectuer un prélèvement de sang placentaire provenant d'un cordon ombilical, un opérateur utilise une aiguille de ponction classique, similaire à celles utilisées pour des dons du sang dans des établissements de transfusion sanguine. Cette technique nécessite que l'opérateur maintienne manuellement l'aiguille dans le cordon ombilical, pendant toute la durée de la collecte de sang, pour éviter que l'aiguille ne se désengage du cordon, mais aussi pour assurer une bonne orientation de l'aiguille dans le cordon, afin de garantir une bonne collecte de sang.

Compte tenu de cette contrainte, les mains de l'opérateur sont immobilisées et ne peuvent exercer d'autres manoeuvres susceptibles d'améliorer la collecte de sang, telles qu'un massage sus-pubien, une traite du cordon, ou encore une agitation de la poche de recueil.

Par ailleurs, des études statistiques montrent qu'environ 40% des prélèvements de sang de cordons ombilicaux sont inutilisables pour des greffes de cellules souches, car trop peu de sang est collecté.

### DESCRIPTION GENERALE DE L'INVENTION

L'objet de l'invention propose un dispositif d'aide au prélèvement de liquide biologique présent dans une cavité, pouvant être plus particulièrement utilisé dans le cadre d'un prélèvement de sang placentaire dans un cordon ombilical, ledit dispositif étant installé sur une aiguille de ponction, améliorant les conditions de la collecte du liquide biologique et permettant à l'opérateur de disposer de ses deux mains.

L'invention concerne donc essentiellement un dispositif d'aide au prélèvement de liquide biologique présent dans une cavité qui est défini dans la revendication 1.

Ainsi, grâce à l'invention, l'aiguille est arrimée à la cavité via les moyens de fixation. Par conséquent, un opérateur effectuant un prélèvement de liquide biologique au moyen d'une aiguille disposant du dispositif selon l'invention, dispose de ses deux mains pour exercer d'autres manoeuvres.

Outre les caractéristiques qui viennent d'être évoquées dans le paragraphe précédent, le dispositif selon l'invention peut présenter une ou plusieurs caractéristiques complémentaires parmi les suivantes, considérées individuellement ou selon toutes les combinaisons techniquement possibles :
▪ Les moyens de fixation sont une pluralité d'ailettes fixées sur le support. Une disposition adaptée des ailettes permet ainsi de maintenir écartées les parois intérieures de la cavité, et par conséquent d'améliorer les conditions de la collecte de liquide biologique.
▪ Le support et les moyens de fixation sont en métal. Dans ce mode de réalisation, la fabrication du dispositif et la manipulation à effectuer pour prélever le liquide biologique sont plus aisées.
▪ Le support et les moyens de fixation sont en plastique. Dans ce mode de réalisation, les risques d'endommager les parois intérieures de la cavité sont minimisés.

Selon un autre aspect, l'invention concerne une aiguille de ponction comportant le dispositif selon l'invention.

L'invention et ses différentes applications seront mieux comprises à la lecture de la description qui suit et à l'examen des figures qui l'accompagnent.

### BREVE DESCRIPTION DES FIGURES

Les figures ne sont présentées qu'à titre indicatif et nullement limitatif de l'invention. Les figures montrent :
- à la figure 1, une représentation schématique d'un dispositif d'aide au prélèvement de liquide biologique selon un premier mode de réalisation de l'invention, coopérant avec une aiguille de ponction ;
- à la figure 2, une représentation schématique du dispositif d'aide au prélèvement de liquide biologique selon un deuxième mode de réalisation de l'invention, avant une ponction ;
- à la figure 3, une représentation schématique du dispositif de la figure 2, après la ponction ;
- à la figure 4, une représentation schématique du dispositif d'aide au prélèvement de liquide biologique selon un troisième mode de réalisation de l'invention, avant une ponction ;
- à la figure 5, une représentation schématique du dispositif de la figure 4, après la ponction.

### DESCRIPTION DETAILLEE D'AU MOINS UN MODE DE REALISATION DE L'INVENTION

Sauf précision contraire, un même élément apparaissant sur des figures différentes présente une référence unique.

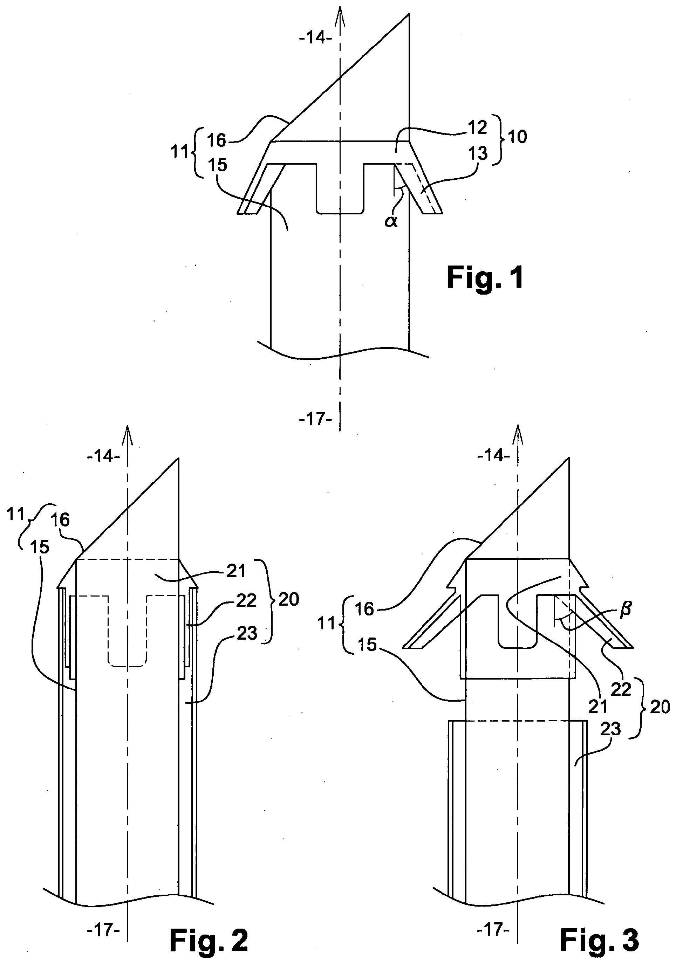

Dans toute la description qui suit, et en référence aux figures 1 à 5, les termes « avant » et « arrière » sont à considérer par rapport à une direction d'avancement d'une aiguille de ponction 11 rencontrée suite à l'introduction de l'aiguille 11 dans une cavité par un opérateur, cette direction étant représentée schématiquement par une flèche 14.

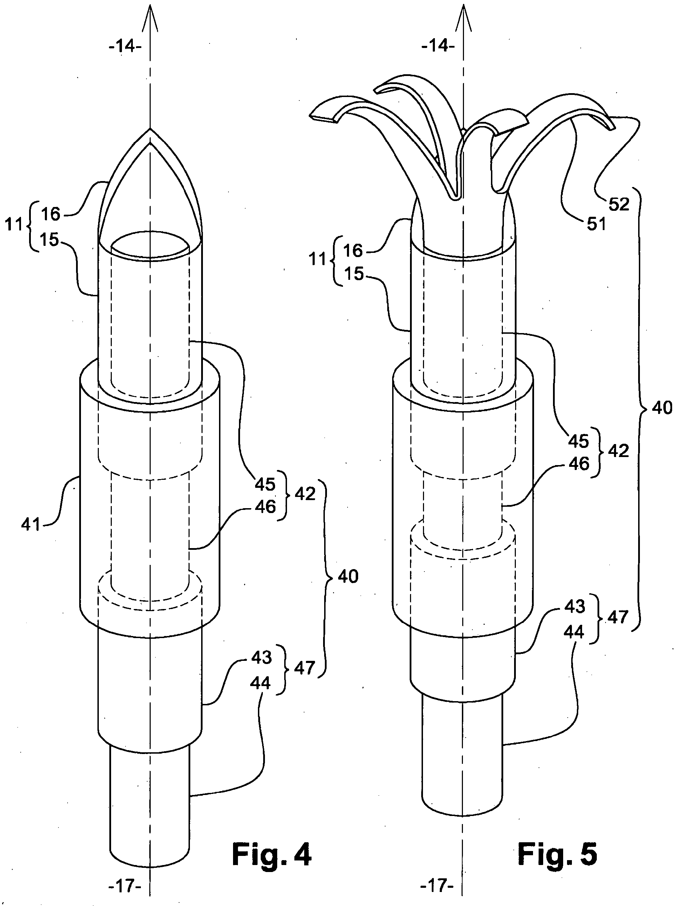

De façon connue, l'aiguille 11 comporte une partie cylindrique 15 et une pointe 16. La partie cylindrique 15 s'étend selon un axe 17, et est surmontée par la pointe 16. Classiquement, la partie cylindrique 15 de l'aiguille 11 est partiellement emboîtée dans un support aiguille 41, représenté aux figures 4 et 5, et non visible aux figures 1 à 3 puisque ledit support aiguille 41 est situé relativement en arrière de l'aiguille 11. Les figures 1 à 3 ne montrent en effet qu'une partie avant de l'aiguille 11. Le support aiguille 41 permet à l'opérateur de tenir l'aiguille 11. Dans le mode de réalisation non limitatif décrit, le support aiguille 41 est de forme cylindrique et s'étend selon l'axe 17. La forme du support aiguille 41 épouse la forme de la partie cylindrique 15 de l'aiguille 11.

La figure 1 est une représentation schématique d'un dispositif d'aide au prélèvement de liquide biologique 10 dans la cavité, ledit dispositif 10 coopérant avec l'aiguille 11, selon un premier mode de réalisation de l'invention.
Le dispositif 10 comporte :
- un support 12
- des moyens de fixation 13
Dans ce premier mode de réalisation :
- Le support 12 est une bague 12 s'étendant selon l'axe 17, et dont la forme épouse la forme de la partie cylindrique 15 de l'aiguille 11. La bague 12 entoure une portion de la partie cylindrique 15 de l'aiguille 11. La hauteur de la bague 12 est de l'ordre de quelques millimètres, par exemple un millimètre. On note que la bague 12 est en contact direct avec la partie cylindrique 15 de l'aiguille 11.
- Les moyens de fixation 13 sont des ailettes 13. Elles sont au nombre de quatre, dont trois sont visibles sur la figure 1, mais selon d'autres modes de réalisation de l'invention, le nombre d'ailettes 13 peut être différent. La longueur des ailettes 13 est de l'ordre de quelques millimètres, par exemple deux millimètres. Les ailettes 13 s'évasent depuis la bague 12 vers l'arrière, c'est-à-dire qu'elles forment un angle α inférieur à 90° avec la surface de l'aiguille 11, et s'étendent dans la direction opposée à la pointe 16 de l'aiguille 11. α est avantageusement compris entre 25° et 35°.

Un tel évasement des ailettes 13 permet une introduction de l'aiguille 11 dans la cavité, mais empêche un retrait de l'aiguille 11 de la cavité. En effet, les ailettes 13 s'évasant vers l'arrière, elles empêchent un mouvement de retrait de l'aiguille 11 selon le principe du harpon : les ailettes 13 s'arriment aux parois intérieures de la cavité. On note que les ailettes 13 sont avantageusement constituées d'un matériau suffisamment souple pour que l'angle α puisse varier de quelques degrés, facilitant ainsi l'introduction de l'aiguille 11 dans la cavité, mais suffisamment rigide pour éviter que les ailettes 13 ne se retournent vers l'avant de l'aiguille 11, c'est-à-dire que l'angle α ne devienne supérieur à 90°. En effet, un retournement des ailettes 13 permettrait un désengagement de l'aiguille 11 de la cavité, or le dispositif 10 est adapté pour rester arrimé aux parois intérieures de la cavité après introduction de l'aiguille 11. Le dispositif 10 est en effet préférentiellement dédié au prélèvement du sang placentaire provenant de cordons ombilicaux, lesdits cordons ombilicaux étant non innervés et condamnés à être coupés après le prélèvement.

Selon un mode de réalisation de l'invention, les ailettes 13 et la bague 12 sont en métal. L'aiguille 11, la bague 12 et les ailettes 13 sont par exemple réalisés d'un seul tenant, ou encore fixées les unes aux autres par soudure. Selon un autre mode de réalisation de l'invention, les ailettes 13 et la bague 12 sont en plastique. La bague 12 est alors par exemple fixée à l'aiguille 11 par collage.

Par ailleurs, la bague 12 est avantageusement installée le plus en avant possible de la partie cylindrique 15 de l'aiguille 11. Dans un mode de réalisation préféré, les ailettes 13 sont installées à une distance comprise entre huit et dix millimètres de la pointe 16 de l'aiguille 11. Ainsi, de par leur forme évasée, les ailettes 13 écartent les parois intérieures de la cavité au plus près de la collecte de liquide biologique par l'aiguille 11. La collecte de liquide biologique est ainsi améliorée.

Les figures 2 et 3 sont des représentations schématiques d'un dispositif d'aide au prélèvement de liquide biologique 20 dans la cavité, ledit dispositif 20 coopérant avec l'aiguille 11, selon un deuxième mode de réalisation de l'invention.

Le dispositif 20 comporte :
- un support 21
- des moyens de fixation 22
- des moyens de maintien 23
Dans ce deuxième mode de réalisation, le support et les moyens dé fixation sont en plastique, et :
- Le support 21 est une bague 21 d'étendant selon l'axe 17, et dont la forme épouse la forme de la partie cylindrique 15 de l'aiguille 11. Le support 21 entoure une portion de la partie cylindrique 15 de l'aiguille 11.
- Les moyens de fixation 22 sont des ailettes 22 solidaires de la bague 21. Les ailettes 22 sont aptes à prendre deux positions :
   ∘ une position rétractée selon laquelle les ailettes 22 sont maintenues contre la partie cylindrique 15 de l'aiguille 11 par les moyens de maintien 23. Cette position rétractée est représentée à la figure 2.
   ∘ une position déployée selon laquelle les ailettes 22 s'évasent depuis la bague 21 vers l'arrière selon un angle β, de sorte qu'elles sont aptes à venir en contact avec la paroi intérieure de la cavité. Cette position déployée est représentée à la figure 3.
- Les moyens de maintien 23 sont un tube de recouvrement 23 en plastique, de forme cylindrique, et s'étendant selon l'axe 17. La forme du tube de recouvrement 23 épouse la forme de la partie cylindrique 15 l'aiguille 11. Le tube de recouvrement 23 est apte à se déplacer le long de la partie cylindrique 15 de l'aiguille 11. Dans la position rétractée, le tube de recouvrement 23 recouvre les ailettes 22, les maintenant plaquées contre la partie cylindrique 15 de l'aiguille 11. L'opérateur insère donc facilement l'aiguille 11 dans la cavité. Puis l'opérateur n'a qu'à faire coulisser le tube de recouvrement 23 vers l'arrière, pour faire passer les ailettes 22 de la position rétractée à la position déployée. L'aiguille 11 ne peut alors plus être retirée de la cavité, les ailettes 22 s'arrimant aux parois intérieures de la cavité selon le principe du harpon.

Le dispositif 20 selon le deuxième mode de réalisation de l'invention est plus compliqué à fabriquer et à manipuler que le dispositif 10 selon le premier mode de réalisation de l'invention, mais l'introduction de l'aiguille 11 dans la cavité est plus aisée, et les risques de créer des lésions sur les parois intérieures de la cavité sont minimisés.

Les figures 4 et 5 sont des représentations schématiques d'un dispositif d'aide au prélèvement de liquide biologique 40 dans la cavité, ledit dispositif 40 coopérant avec l'aiguille 11, selon un troisième mode de réalisation de l'invention.
Le dispositif 40 comporte :
- un support 42
- des moyens de fixation 51
- des moyens d'actionnement 47
Dans ce troisième mode de réalisation :
- le support 42 est un cathéter 42. Le cathéter 42 est de forme cylindrique et s'étend selon l'axe 17. Le cathéter 42 comporte une partie supérieure 45 contenue dans l'aiguille 11, et une partie inférieure 46 contenue dans le support aiguille 41. Le cathéter 42 est apte à se déplacer selon l'axe 17, via les moyens d'actionnement 47.
- Les moyens de fixation 51 sont des ailettes 51. Elles sont au nombre de quatre, mais selon d'autres modes de réalisation de l'invention, le nombre d'ailettes peut être différent.
- Les moyens d'actionnement 47 comportent une pièce de connexion 43 et une pièce de vissage 44. La pièce de connexion 43 et la pièce de vissage 44 sont de forme cylindrique et s'étendent selon l'axe 17. La pièce de connexion 43 est fixée d'un côté à la partie inférieure 46 du cathéter 42, et d'un autre côté à la pièce de vissage 44. La pièce de connexion 43 est apte à se déplacer par vissage le long de la pièce de vissage 44, entrainant dans son mouvement le cathéter 42 dont elle est solidaire.
Les ailettes 51 sont aptes à prendre deux positions :
- une position rétractée selon laquelle les ailettes 51 sont maintenues contre une paroi intérieure de l'aiguille 11. Cette position rétractée est représentée à la figure 4.
- une position déployée selon laquelle les ailettes 51 s'évasent depuis le cathéter 42 vers l'avant, c'est-à-dire qu'elles forment un angle γ inférieur à 90° avec la surface de l'aiguille 11, et s'étendent en direction de la pointe 16 de l'aiguille 11. En position déployée, les ailettes 51 viennent en contact avec la paroi intérieure de la cavité. Cette position déployée est représentée à la figure 5. Chaque ailette 51 dispose d'une extrémité 52 recourbée vers l'arrière, de sorte que l'ailette 51 soit apte à s'arrimer à la paroi intérieure de la cavité selon le principe du harpon.

Le passage d'une position à une autre s'opère via les moyens d'actionnement 47 : en vissant vers l'avant la pièce de connexion 43 le long de la pièce de vissage 44, l'opérateur provoque un déplacement du cathéter 42 vers l'avant. Les ailettes 51 passent alors de la position rétractée à la position déployée, en sortant de l'aiguille 11. Les ailettes 51 s'arriment alors à la paroi intérieure de la cavité, empêchent ainsi un retrait de l'aiguille 11 hors de la cavité. On note que le passage d'une position à l'autre s'effectue sans ouverture du circuit de prélèvement, ce qui permet un prélèvement stérile du liquide biologique. Par ailleurs, de par leur forme évasée, les ailettes 51 écartent les parois intérieures de la cavité améliorant ainsi la collecte de liquide biologique.

## Revendications

1. Dispositif (20) d'aide au prélèvement de liquide biologique présent dans une cavité, ledit dispositif (20) étant apte à coopérer avec une aiguille de ponction (11), ledit dispositif (20) étant **caractérisé en ce qu'**il comporte :
- un support de maintien (21) apte à maintenir solidaire ledit dispositif (20) et ladite aiguille (11) lors de l'introduction de l'aiguille (11) dans la cavité, ledit support (21) étant une bague (21) apte à entourer au moins une portion de l'aiguille (11) en épousant la forme extérieure de l'aiguille (11)
- des moyens de fixation (22) aptes à se fixer, après introduction de l'aiguille (11) dans la cavité, sur une paroi intérieure de la cavité
- des moyens de maintien (23), tels que les moyens de fixation (22) sont aptes à prendre deux positions :
∘ une position rétractée selon laquelle les moyens de fixation (22) sont maintenus rétractés par les moyens de maintien, de sorte qu'ils sont aptes à être maintenus contre la paroi cylindrique de l'aiguille (11),
∘ une position déployée selon laquelle les moyens de fixation (22) s'évasent depuis le support (21) lors du retrait des moyens de maintien (23), de sorte qu'ils sont aptes à venir en contact avec la paroi intérieure de la cavité.
les moyens de maintien (23) étant un tube de recouvrement apte à recouvrir au moins en partie la paroi extérieure de l'aiguille (11), ledit tube de recouvrement (23) étant apte à se déplacer le long de l'aiguille (11).

2. Dispositif (20) selon la revendication précédente, **caractérisé en ce que** les moyens de fixation (22) sont une pluralité d'ailettes (22) fixées sur le support (21).

3. Dispositif (20) selon l'une des revendications précédentes, **caractérisé en ce que** le support (21) et les moyens de fixation (22) sont en métal.

4. Dispositif (20) selon l'une des revendications 1 à 2, **caractérisé en ce que** le support (21) et les moyens de fixation (22) sont en plastique.

5. Aiguille de ponction (11) comportant le dispositif (20) selon l'une des revendications 1 à 4.

## Patentansprüche

1. Hilfsvorrichtung (2) zur Entnahme von in einer Aushöhlung vorhandener biologischer Flüssigkeit, wobei die genannte Vorrichtung (20) geeignet ist, mit einer Punkturnadel (11) zusammenzuwirken, wobei die genannte Vorrichtung (20) **dadurch gekennzeichnet ist, dass** sie umfasst:
- eine Haltestütze (21), die geeignet ist, die genannte Vorrichtung (20) und die genannte Nadel (11) beim Einführen der Nadel (11) in die Aushöhlung fest verbunden zu halten, wobei die genannte Stütze (21) ein Ring (21) ist, der geeignet ist, wenigstens einen Abschnitt der Nadel (11) unter Annahme der äußeren Form der Nadel (11) zu umgeben
- Befestigungsmittel (22), die geeignet sind, sich nach dem Einführen der Nadel (11) in die Aushöhlung auf einer inneren Wand der Aushöhlung zu fixieren
- Haltemittel (23), wie z. B. Befestigungsmittel (22), die geeignet sind, zwei Positionen einzunehmen:
∘ eine eingezogene Position, nach der die Befestigungsmittel (22) durch Haltemittel derart eingezogen gehalten werden, dass sie geeignet sind, gegen die zylindrische Wand der Nadel (11) gehalten zu werden,
∘ eine eingezogene Position, nach der die Befestigungsmittel (22) sich ab dem Träger (21) beim Zurückziehen der Haltemittel (23) derart aufweiten, dass sie geeignet sind, mit der inneren Wand der Aushöhlung in Kontakt zu treten,
wobei die Haltemittel (23) eine Abdeckröhre sind, die geeignet ist, wenigstens zum Teil die äußere Wand der Nadel (11) abzudecken, wobei die genannte Abdeckröhre (23) geeignet ist, sich entlang der Nadel (11) zu verschieben.

2. Vorrichtung (20) gemäß dem voranstehenden Anspruch, **dadurch gekennzeichnet, dass** die Befestigungsmittel (22) eine Vielzahl aus kleinen Flügeln (22) sind, die auf dem Träger (21) befestigt ist.

3. Vorrichtung (20) gemäß einem der voranstehenden Ansprüche, **dadurch gekennzeichnet, dass** der Träger (21) und die Befestigungsmittel (22) aus Metall sind.

4. Vorrichtung (20) gemäß Anspruch 1 bis 2, **dadurch gekennzeichnet, dass** der Träger (21) und die Befestigungsmittel (22) aus Plastik sind.

5. Punkturflügel (11), umfassend die Vorrichtung (20) gemäß Anspruch 1 bis 4.

## Claims

1. A device (20) for assisting with the sampling of biological liquid present in a cavity, said device (20) being able to cooperate with a puncture needle (11), said device (20) being **characterised in that** it includes:
- a holding support (21) able to hold said device (20) and said needle (11) attached upon introducing the needle (11) into the cavity, said support (21) being a ring (21) able to surround at least a portion of the needle (11) by fitting closely to the outer shape of the needle (11)
- fixing means (22) able to fix, after introducing the needle (11) into the cavity, on an inner wall of the cavity
- holding means (23), such that the fixing means (22) are able to adopt two positions:
∘ a retracted position according to which the fixing means (22) are held retracted by the holding means, so as to be able to be held against the cylindrical wall of the needle (11),
∘ an extended position according to which the fixing means (22) flare out from the support (21) upon withdrawing the holding means (23), so as to be able to contact the inner wall of the cavity,
the holding means (23) being a covering tube able to at least partly cover the outer wall of the needle (11), said covering tube (23) being able to move along the needle (11).

2. The device (20) according to the preceding claim, **characterised in that** the fixing means (22) are a plurality of fins (22) fixed to the support (21).

3. The device (20) according to one of the preceding claims, **characterised in that** the support (21) and the fixing means (22) are made of metal.

4. The device (20) according to one of claims 1 to 2, **characterised in that** the support (21) and the fixing means (22) are made of plastic.

5. A puncture needle (11) including the device (20) according to one of claims 1 to 4.
